# EUROPÄISCHE PATENTSCHRIFT

(11) **EP 1 528 395 B1**
(45) Veröffentlichungstag und Bekanntmachung des Hinweises auf die Patenterteilung: **23.04.2014**
(21) Anmeldenummer: 04025025.0
(22) Anmeldetag: 21.10.2004
(51) Int. Cl.: G01N 33/564, G01N 33/68

(54) **Diagnostizierung von Erkrankungen und Prognostizierung von deren Verläufen über T-Helferzellen**
Diagnosis and prognosis of disease states via T-helper cells
Diagnostic de maladies et prognostic par le moyen de cellules T

(30) Priorität: 03.11.2003 DE 10352678
(43) Veröffentlichungstag der Anmeldung: 04.05.2005
(73) Patentinhaber: Autoimmun Diagnostika GmbH, 72479 Strassberg (DE)
(72) Erfinder: Schöllhorn, Volkmar, Dr., 72458 Albstadt (DE)
(74) Vertreter: Mütschele, Thomas

(56) Entgegenhaltungen:
- EP-A- 0 957 359
- EP-A- 1 262 777
- DE-A1- 10 029 496
- US-A- 6 022 697
- US-A1- 2003 003 485
- OGAWARA H. ET AL.: "High Th1/Th2 ratio in patients with chronic idiopathic thrombocytopenic purpura." EUROPEAN JOURNAL OF HAEMATOLOGY, Bd. 71, Nr. 4, Oktober 2003 (2003-10), Seiten 283-288, XP002355035 ISSN: 0902-4441
- CANETE J.D. ET AL.: "Differential Th1/Th2 cytokine patterns in chronic arthritis: Interferon gamma is highly expressed in synovium of rheumatoid arthritis compared with seronegative spondyloarthropathies" ANNALS OF THE RHEUMATIC DISEASES, Bd. 59, Nr. 4, April 2000 (2000-04), Seiten 263-268, XP002355116 ISSN: 0003-4967

## Beschreibung

Die Erfindung betrifft ein Verfahren zur Diagnostizierung von Erkrankungen bzw. zur Prognostizierung von deren Verläufen, sowie ein diesbezügliches Kit.

Bei allen Erkrankungen werden antigenspezifische T-Zellen gebildet. Die wichtigsten stellen die T-Helferzellen dar. Sie sind die zentralen Steuerungseinheiten für alle Komponenten des Immunsystems. Diese T-Helferzellen teilen sich in zwei Subtypen auf. Die T_{H1}-Zellen bilden die eher aktivierenden proinflammatorischen Zellen, während die T_{H2}-Zellen (häufig auch als Suppressorzellen bezeichnet) die eher suppremierenden Komponenten, d. h. das Immunsystem eher hemmend, darstellen. Die T_{H1}-Helferzellen aktivieren dabei nach Kontakt mit antigen-präsentierenden Zellen Makrophagen und Killerzellen und fördern damit die klonale Vermehrung und Reifung von spezifisch sensibilisierten B-Lymphozyten zu Plasmazellen sowie die Bildung von spezifischen Antikörpern. Die stimulierenden Effekte werden unter anderem durch antigenspezifische oder unspezifische humorale Faktoren (Lymphokine und Interferone), zum Teil durch direkten Zellkontakt vermittelt.

Für die Regulation bzw. Modulation der Immunantwort sind neben den Helferzellen die Suppressorzellen bedeutsam. Diese unterdrücken die Immunantwort anderer T- und B-Lymphozyten. Hierfür setzen sie nach Stimulation durch ein spezifisches Antigen humorale Faktoren frei, die wahrscheinlich auf die antigenpräsentierenden Zellen übertragen werden und die Aktivität anderer Lymphozyten mit entsprechender Spezifität bei Kontakt mit den antigenpräsentierenden Zellen hemmen. Somit stellen die Helfer- und Supressorzellen (nachfolgend als T-Helferzellen bezeichnet) ein ausgeklügeltes System dar, mit dessen Hilfe das Immunsystem auf äußere Faktoren (wie z. B. das Eindringen von Krankheitserregern) stimulierend (durch Aktivierung der T-Helferzellen) bzw. supprimierend (durch Aktivierung der Suppressorzellen) reagieren kann.

Diese T-Helferzellen-Subtypen unterscheiden sich unter anderem dadurch, daß sie unterschiedliche Zytokinprofile aufweisen. Unter Zytokine werden allgemein sämtliche Substanzen erfaßt, die von Zellen (z. B. T-Helferzellen) produziert werden, um auf externe Stimuli zu reagieren. Darunter fallen z. B. die Lymphokine, Interleukine und Monokine.

Lymphokine sind die Substanzen, die von Lymphozyten (vor allem antigenaktivierten T-Lymphozyten) produziert und sezerniert werden und andere Zellen aktivieren oder deren Funktionen beeinflussen sowie zur Bildung verschiedener Enzyme und anderer Faktoren (z. B.

Sauerstoffmetabolite) oder zur Proliferation anregen. Wichtige Vertreter dieser Substanzklasse sind z. B. Hautreaktiver Faktor, Migrationshemmfaktor, Makrophagenaktivierender Faktor, Chemotaktischer Faktor, Koloniestimulierender Faktor, Mitogene Faktor, Lymphotoxine, Interferone etc. Interleukine sind von Leukozyten sezernierte Signalsubstanzen der Immunregulation. Bekannte Vertreter sind Interleukin-2 (II-2), Interleukin-3 (II-3), Interleukin-4 (II-4) sowie Interleukin-10 (II-10). II-2 wird beispielsweise von T-Helferzellen produziert und aktiviert T-Lymphozyten und Killerzellen. Gentechnologisch hergestelltes rekombinantes II-2 wurde mit Erfolg in der Therapie von Nierenkarzinomen und malignen Melanomen angewendet. Monokine sind Substanzen, die von Monozyten und anderen Zellen (die gleichen, die auch Interleukine herstellen können) produziert werden und die Funktion anderer Zellen beeinflussen (z. B. spezifische und unspezifische Lymphozyten-aktivierende Faktoren, Wachstumsfaktoren). All diese Substanzen, die wie schon dargelegt, unter dem Oberbegriff Zytokine zusammengefaßt werden, zeichnen sich durch eine Gemeinsamkeit aus. Sie beeinflussen das Immunsystem.

Bei den gegenwärtigen Diagnostizierungen bzw. Prognostizierungen von Krankheiten und deren Verläufen steht immer mehr nicht nur die Erkrankung selbst im Vordergrund (z. B. Infektionserkrankung, Autoimmunerkrankung, Allergie etc.), sondern vielmehr, ob diese Erkrankung von selbst ausheilt, d. h. ohne spezielle Therapie (z. B. Zugabe von Medikamenten) von dem Immunsystem selbst erfolgreich bekämpft werden kann, sowie die Erkenntnis, inwiefern die Erkrankung in ein akutes oder chronisches Stadium übergeht. Chronische Krankheitsstadien haben meistens schwere Schädigungen von Organen zur Folge. So führt z. B. eine chronische Hepatitis C, d. h. eine progredient verlaufende Leberentzündung verursacht durch Hepatitis-C Viren, häufig zu einer Leberfibrose oder Leberzirrhose. Ursächlich ist dabei wahrscheinlich eine Insuffizienz des zellulären Immunsystems, d. h. die Fortdauer gestörter Immunmechanismen scheint ursächlich eine Rolle bei dieser Erkrankung zu spielen.

Generell stellt sich dem Praktiker (d. h. dem im Gesundheitssystem Tätigen wie z. B. Mediziner, Laborpersonal etc.) die Aufgabe, das Krankheitsstadium richtig zu diagnostizieren sowie eine verlässliche Prognose über deren weiteren Verlauf abzugeben. Dies ist insofern wichtig, als bei einer guten Prognose die Selbstheilungsmechanismen des Immunsystems ausreichen, um die Erkrankung zu bekämpfen und somit das Immunsystem zu stärken. Zusätzliche Therapiemaßnahmen sind dann nicht nötig, führen diese doch häufig zu einer Belastung des Patienten, z. B. durch Medikamentenzugabe und damit einhergehende Reaktionen wie z. B. Resistenzen, Überempfindlichkeitsreaktionen, schädliche Metaboliten etc.

Gegenwärtig stellt sich die Situation für den Praktiker häufig dahingehend dar, daß bei ersten Anzeichen einer Erkrankung (z. B. bei Reaktion des Körpers auf Fremdantigene durch Bildung von Antikörpern) erste Therapiemaßnahmen eingeleitet werden, ohne auf die individuelle Prädisposition des Patienten oder den "Schweregrad" der Erkrankung einzugehen.

Wünschenswert wäre es, Krankheitsstadien spezifisch diagnostizieren bzw. deren Verläufe prognostizieren zu können, um so die bestmögliche Therapie (welche auch gar keine Therapie bedeuten kann) dem Patienten angedeihen lassen zu können.

Aus der EP 0 957 359 A2 ist ein ELISPOT-Verfahren zur Bestimmung der Aktivität von menschlichen und tierischen Zellen bekannt.

Gegenstand der US 6,022,697 ist ein Verfahren zur Detektion des Status einer insulinabhängigen Diabetes Mellitus-assoziierten Autoimmunantwort, bei welchem das Verhältnis von T_{H1}-Zellen zu T_{H2}-Zellen bestimmt wird.

Weiterhin ist die Bestimmung des Verhältnisses von T_{H1}-Zellen zu T_{H2}-Zellen im Zusammenhang mit der idiopathischen thrombocytopenischen Purpura (ITP) bekannt (Ogaware et al.: High Th1/Th2 ratio in patients with chronic idiopathic thrombocytopenic purpura; Eur J Haematol 2003: 71 : 283-288).

Ebenso wurden bereits T_{H1}-/T_{H2}-Cytokinmuster im Zusammenhang von chronischer Arthritis untersucht (Cañete et al.: Differential Th1/Th2 cytokine patterns in chronic arthritis: interferon γ is highly expressed in synovium of rheumatoid arthritis compared with seronegative spondyloarthropathies; Ann Rheum Dis 2000; 59:263-268).

Ein Verfahren zur Identifizierung von Antigenen unter Verwendung der ELISPOT-Technologie ist aus der US 2003/0003485 A1 bekannt.

Ein Verfahren zur quantitativen Bestimmung der Spezifität und Aktivität von T-Zellen aus humanem Untersuchungsmaterial auf Basis des ELISPOT-Verfahrens ist Gegenstand der DE 100 29 496 A1.

Diese Aufgabe wird gelöst durch die Bereitstellung eines Verfahrens, wie es in Anspruch 1 beschrieben wird. Bevorzugte Ausführungsformen sind in den Ansprüchen 2 bis 11 dargestelft. Der Wortlaut sämtlicher Ansprüche wird hiermit durch Bezugnahme zum Inhalt der Beschreibung gemacht.

Überraschenderweise wurde gefunden, daß die Diagnostizierung eines Krankheitsstadiums bzw. die Prognose über den Verlauf einer Erkrankung von dem Verhältnis der T_{H1}- zu T_{H2}-Zellen abhängt. Generell kann man eine allgemeine Aussage dahingehend treffen, daß die Menge an T_{H2}-Zellen entscheidend für die Heilungschancen sind. Je mehr T_{H2}-Zellen (im Verhältnis zu T_{H1}-Zellen), um so langwieriger ist die Ausheilung der Erkrankung und umso mehr sind Therapiemaßnahmen angesagt. Abhängig von der Erkrankung, die untersucht wurde, konnte festgestellt werden, daß eine große Zahl (d. h. viele T_{H1-}Zellen im Verhältnis zu T_{H2-}Zellen) eine gute Prognose für die untersuchte Erkrankung widerspiegelt, während eine kleine Zahl (d. h. viele T_{H2}-Zellen im Verhältnis zu T_{H1}-Zellen), eine schlechtere bis schlechte Prognose wiedergibt.

Erfindungsgemäß wird daher ein Verfahren zur Diagnostizierung von Erkrankungen und/oder zur Prognostizierung von deren Verläufen beansprucht, wobei die Anzahl von T_{H1}- und T_{H2}-Zellen (T-Helferzellen) zellulär produzierten Zytokinen in vom lebenden Organismus entnommenen menschlichen oder tierischen Zellen gemessen werden, und anschließend das Verhältnis von T_{H1}- zu T_{H2}-Zellen zellulär produzierten Zytokinen bestimmt wird.

Die Anzahl von T_{H1}- und T_{H2}-Zellen (T-Helferzellen) zellulär produzierten Zytokine wird mittels der ELISA-Spot-Methode (Elispot-Methode) ermittelt. Die Elispot-Methode ist wesentlich empfindlicher als der ELISA-Test oder die FACS-Analyse, die gegenwärtig gängige Diagnoseverfahren darstellen. Die Elispot-Methode kann eine spezifische Zelle aus über einer Million unspezifischer Zellen herausfinden. Daher kann der Test sowohl für die Früherkennung bzw. Diagnostizierung von Erkrankungen, z. B: die Früherkennung von infizierten Patienten, als auch für das Monitoring, d. h. der Überwachung von erkrankten Patienten und damit einhergehend der Prognostizierung von Erkrankungsverläufen bzw. der Therapierung von Erkrankungen, verwendet werden. Die Früherkennung, das Monitoring sowie die Prognostizierung ist sowohl bei Menschen als auch beim Tier möglich.

Weiterhin zeichnet sich das Verfahren dadurch aus, daß die von den T-Helferzellen zellulär produzierten Zytokine in Gegenwart von stoffspezifischen Fremdstoffen inkubiert werden, wobei die produzierten Zytokine mit den stoffspezifischen Fremdstoffen interagieren, und diese Interaktion durch eine Farbreaktion nach der ELISA-Spot-Methode nachgewiesen wird. Eine geeignete Farbreaktion für die Elispot-Methode stellt z. B. die Lumineszenz- oder die Fluoreszenzfärbung dar.

Der Vorteil der Elispot-Methode besteht darin, daß zum Nachweis von erkrankten Zellen nicht nur die Anzahl der reaktiven Zellen gemessen wird, um daraus Rückschlüsse auf den Grad der Erkrankung ziehen zu können, sondern vor allem die Qualität der Reaktion einzelner Zellen bestimmt wird. So können einzelne Zellen sehr unterschiedlich auf gleiche Reizstoffe reagieren, was sich in der Quantität der abzugebenden bzw. zu detektierenden Zytokine und damit in den örtlichen Quantitäten der Farbreaktion jeder einzelnen Zelle ausdrückt. Es ist daher vorteilhaft, nicht nur die Anzahl der reaktiven Zellen zu ermitteln, sondern die Intensität der Gesamtreaktion zu bestimmen. Dieses erlaubt wesentlich weitergehende Schlüsse auf den Grad der Erkrankung einer Zelle bzw. höhere Sensitivitäten, falls der Nachweis einer Erkrankung erbracht werden soll. So ist es möglich, mit Hilfe der Elispot-Methode und verschiedener Elispot-Variationen wesentlich zuverlässigere und aussagekräftigere Auswertungen durchzuführen. So können, um ein exaktes T-Helferzellenverhältnis bestimmen zu können, die einzelnen zellular produzierten Zytokine für jede auf ein spezifisches Antigen reagierende Zelle ermittelt werden. Diese (semiquantitative) Messung kann anhand der Qualität des Spots erfolgen. Dabei ist die Qualität des Spots abhängig von der Fläche und der Intensität des untersuchten Spots. Bei solch einer Auswertung ergibt sich typischerweise eine Glockenkurvenverteilung, wobei die Mengen/Flächen unterhalb der Kurve den jeweiligen zellulär produzierten Zytokine entsprechen.

Gemäß einer weiteren bevorzugten Ausführungsform der Erfindung handelt es sich bei den menschlichen oder tierischen Zellen um Blutzellen. Die Blutzellen können vor der Messung an T-Helferzellen, von diesen zellulär produzierten Zytokinen und/oder vor der Inkubation angereichert werden, um so die Sensitivität und Spezifität des Nachweises zu erhöhen. Bei den Blutzellen handelt es sich um Lymphozyten, insbesondere um T-Zellen, die für das erfindungsgemäße Verfahren benutzt werden können.

Bei dem von T_{H1}-Zellen produzierten Zytokin handelt es sich um γ-Interferon. Das von T_{H2}-Zellen produzierte Zytokin ist Interleukin-10. Es wird das Verhältnis von γ-Interferon als Marker für T_{H1}-Zellen und Interleukin-10 als Marker für T_{H2}-Zellen bestimmt. Es kann sich dabei generell um eine semi- bzw. quantitative Bestimmung der von den T-Helferzellen zellulär produzierten Zytokine handeln. Es hat sich in der praktischen Arbeit gezeigt, daß die besten Ergebnisse erzielt werden, wenn die Verhältnisse von γ-Interferon als Marker von TH₁₋Zellen und Interleukin-10 als Marker für T_{H2}-Zellen bestimmt werden. Nach Messung der von den T-Helferzellen zellulär produzierten Zytokine und Bestimmung des Verhältnisses der Markerstoffe für diese Zellen kann dann eine Aussage getroffen werden, ob die jeweilige Erkrankung einen chronischen Verlauf haben wird bzw. selbst ausheilt. In diesem Zusammenhang ist es wichtig darauf hinzuweisen, daß das Verhältnis nur anhand der jeweiligen spezifischen (antigenspezifischen) Zellen bestimmt werden soll. So sollen bei einer Infektion (z. B. eine Borreliose) nur die T-Helferzellen erfaßt werden, die auch gegen verschiedene Borrelienantigene reagieren und nicht generell alle T-Helferzellen, die erfaßt werden können, da diese z. B. gegen andere Erreger (z. B. Allergene, andere Infektionen etc.) gerichtet sein können und das Ergebnis verfälschen würden. Hierbei tritt generell ein methodisches Problem auf. Die antigenspezifischen Zellen sind häufig so selten, daß sie selbst mit der Elispot-Methode manchmal nur grenzwertig erfaßbar sind. So findet man z. B. bei einer normalen Borrelioseinfektion bei ca. 100.000 peripheren blutmononuklearen Zellen (PBMCs) nur ca. 20 bis 200 borrelienspezifische T-Helferzellen vom Typ 1 (erfaßt anhand der γ-Interferonproduktion). Beim gleichen Probanden sind nur 0 bis 10 Zellen zu erfassen, die borrelienspezifische Zellen vom T_{H2}-Typ liefern (gemessen anhand der II-4 oder II-5 Produktion). Bei diesen relativ kleinen Werten kann keine statistisch relevante Aussage über die Verhältnisse von T_{H1} zu T_{H2} zellulär produzierte Zytokine abgegeben werden. Ebenso hat sich gezeigt, daß die II-4 oder II-5 Produktion bei Borreliose, d. h. der Nachweis an diesen Interleukinen, stark von dem benutztem Substrat abhängt. Es hat sich gezeigt, daß der Vergleich von γ-Interferon und II-10 bei einer Borreliose und einer Direktinkubation, d. h. ohne vorhergehende Vorinkubation und/oder Stimulierung der Zellen, einige Vorteile hat. Man erhält auswertbarere Anzahlen an antigenspezifischen Zellen. So wurden z. B. 50 γ-Interferon und 300 Interleukin-10 Spots bei den Probanden gemessen. Diese Zahlen können statistisch sinnvoll bearbeitet werden und ergeben ein aussagefähigeres Verhältnis zwischen T_{H1}- und T_{H2}-Zellen. Es kann hierbei auch berücksichtigt werden, daß II-10 auch von anderen Zellen als nur T_{H2}-Zellen gebildet werden kann. Bei Vorinkubation des Ansatzes und Messung erst nach Stunden in der Elispotplatte sehen die Ergebnisse für eine Borrelioseinfektion etwas anders aus, da viele Zellen adhärieren und nicht mehr auf die Platte überführt werden können. Somit ist zumindest für Borrelioseinfektionen eine sofortige Inkubation, ohne vorhergehende Vorinkubation, und Bestimmung des Verhältnisses von γ-Interferon zu Interleukin-10 besonders bevorzugt. In den meisten Fällen kann Interleukin-10 als Marker für T_{H2}-Zellen besonders gut verwendet werden, da dieses Zytokin häufiger detektiert werden kann (unabhängig von der zu untersuchenden Erkrankung) und somit für statistisch relevante Aussagen eher zu verwenden ist.

In einer bevorzugten Ausführungsform handelt es sich bei den Erkrankungen, die diagnostiziert und/oder deren Verläufe prognostiziert werden sollen, um Infektionserkrankungen, Autoimmunerkrankungen und/ oder Allergien. Diese können durch eine Farbreaktion mit für diese Erkrankungen spezifischen Fremdstoffen nachgewiesen werden. Es existieren z. B. eine Reihe von spezifischen Antigenen und Antikörpern, die dazu verwendet werden können. Bei den Infektionserkrankungen werden insbesondere Borreliose, CMV (controlled mechanical ventilation; Zytomegalie-Virus), EBV (Eppstein-Barr-Virus), Herpes, Hepatitis C und/ oder Tuberkulose von der Erfindung umfaßt. Generell können aber auch alle anderen Infektionserkrankungen, die mit oder ohne Hilfe der Elispot-Methode im Blut nachgewiesen werden können, erfindungsgemäß diagnostiziert und/oder prognostiziert werden. Sofern es sich bei der Erkrankung um eine Autoimmunerkrankung handelt, sind erfindungsgemäß Erkrankungen wie Rheuma und/oder Morbus-Crohn zu nennen. Wie aber schon dargelegt, werden generell alle Erkrankungen umfaßt, die mit dem erfindungsgemäßen Verfahren diagnostiziert und/oder prognostiziert werden können.

In einer weiteren bevorzugten Ausführungsform handelt es sich bei den stoffspezifischen Fremdstoffen um sogenannte Capture-Moleküle, insbesondere um Antigene und/oder Antikörper. Diese stoffspezifischen Fremdstoffe, insbesondere die Antikörper, werden dazu benutzt, um z. B. auf einer Mikrotiterplatte gekoppelt zu werden. Auf diese werden dann später die zu untersuchenden Zellen aufgetragen, so daß die zellulär produzierten Zytokine an örtlich benachbarte Antigene und/oder Antikörper binden und so einen Nachweis ermöglichen. Im allgemeinen können verschiedene zellulär produzierte Zytokine mit verschiedenen zellulär produzierten spezifischen Fremdstoffen spezifisch interagieren, wobei diese vorzugsweise spezifisch mit diesen binden.

In einer bevorzugten Ausführungsform führt die spezifische Interaktion zu mindestens zwei, vorzugsweise zu einer Vielzahl, an Farbreaktionen, wobei diese nach Art der Farbe getrennt erfaßt und ausgewertet werden können. Zum Beispiel ist es möglich, mit Hilfe von verschiedenen Immunfluoreszenzfarbstoffen, Zytokine nachzuweisen bzw. zu ermitteln, die ausgeschieden worden sind. Man kann ferner durch Nutzung von Fluorochromen mit unterschiedlichen Absorptionswellenlängen Mehrfachmarkierungen durchführen.

In einer besonders bevorzugten Ausführungsform der Erfindung werden Zellen in einem Untersuchungsgefäß, insbesondere in den Löchern von Mikrotiterplatten, fixiert. Dort können die von den Zellen produzierten Zytokine auf den Zellen selbst, unter Nutzung geeigneter Nachweisverfahren wie z. B. die oben beschriebenen Farbreaktionen, detektiert werden. Zum Fixieren sind beispielsweise Glutaraldehyd, Formamid, Aceton, Methanol, Ethanol und andere, dem Fachmann bekannte Fixierlösungen (Lösungsmittel), geeignet. Durch diese Fixierung ist es auch möglich, die einzelnen Zellpopulationen phänotypisch voneinander zu unterscheiden, z. B. eine Typisierung von B- und/oder T-Zellen vorzunehmen. In dem nachfolgenden Verfahren kann dann geklärt werden, welche Zellen positiv sind bzw. welche zellulär produzierte Zytokine tragen, enthalten bzw. abgeben. Alternativ dazu können die Zellen vorher lysiert werden. So können die dann abgegebenen zellulär produzierten Zytokine mit Hilfe eines Fremdstoffes weggefangen und nachgewiesen werden, insbesondere in unmittelbarer Umgebung der jeweiligen lysierten Zelle.

In einer weiteren bevorzugten Ausführungsform können die Zellen in einem stimulierten Zustand inkubiert werden. Dies erfolgt vorzugsweise ex *vivo,* aber auch eine *in vivo* Stimulation ist erfindungsgemäß vorgesehen. Als Stimulanzien können vorzugsweise Mitogene und/oder Antigene vorgesehen sein. Mit Hilfe dieser Methoden ist z. B. eine Optimierung des geeigneten Zeitpunktes für die Blutentnahme möglich. Diese Methode kann man sich ferner dazu nutzbar machen, die Immunreaktion des Körpers anhand zahlreicher Immunparameter nach einer Erkrankung zu messen, was Z. B. wichtig ist für die genaue Definition der Konzentration der von den Zellen produzierten Zytokine, die für ein bestimmtes Krankheitsstadium und/oder eine Prognose notwendig sind, d. h. die Frage zu klären, wie viele positive Signale für eine bestimmte Erkrankung nötig sind, um in ein bestimmtes Krankheitsstadium zu geraten oder einen bestimmten Krankheitsverlauf zu durchlaufen. Als Stimulanzien lassen sich z. B. Mitogene wie Con-A einsetzen. Vorzugsweise erfolgt hierbei durch die Stimulierung eine Vermehrung der von den T-Helferzellen zellulär produzierten Zytokine. Dies kann man sich z. B. dafür nutzbar machen, daß zuvor nicht ausreichend vorhandene Mengen an bestimmten Markerstoffen (bzw. zu geringe Mengen, so daß eventuell Aussagen nicht genau genug sind) nach der Stimulierung ausreichend vorhanden sind, um eine valide Aussage treffen zu können.

Ein weiteres mögliches Einsatzgebiet ist das Monitoring, d. h. die Überwachung von Krankheitsverläufen. So kann z. B. bei einer erfolgten Erkrankung eines Patienten diesem Blut entnommen und, sofern nötig, aus diesem die Lymphozyten isoliert und anschließend das T-Helferzellen Verhältnis bestimmt werden. Je nach Ergebnis dieser Bestimmung kann dann ein Therapieplan erstellt werden. So werden bei einem ungünstigen T-Helferzellenverhältnis diese mit spezifischen Antigenen stimuliert. Hierbei können sowohl spezifische Antigene für T_{H1}- als auch für T_{H2}-Zellen zum Einsatz kommen, je nach erwünschter Reaktion. So wird im allgemeinen bei einer Infektionserkrankung (wie z. B. einer Borreliose) eher versucht werden, ein günstiges T_{H1} Verhältnis herzustellen. Nach erfolgter Stimulation mit dem entsprechenden Antigen(en) können die stimulierten (ggf. vermehrten und Borrelienspezifischen)) T_{H1}-Zellen isoliert und anschließend dem Patienten wieder zurückgegeben werden, um so den Krankheitsverlauf positiv zu beeinflussen. Bei Autoimmunerkrankungen kann das gleiche Verfahren angewendet werden, wobei hier eher die T_{H2}-Helferzellen im Vordergrund stehen. Alternativ zur Isolierung der T-Helferzellen können diese auch direkt, d. h. ohne vorherige Isolation, dem Patienten zurückgegeben werden. Weiterhin kann dieses Verfahren zur Prophylaxe gegen bestimmte Erkrankungen oder zur Überwachung des Impfstatus eingesetzt werden. Generell hängen die Inkubationszeiten von der zu untersuchenden Erkrankung und der Menge an T-Helferzellen und/oder der von ihnen produzierten Stoffe ab. Im allgemeinen kann die Inkubationsdauer von ca. 30 Minuten bis ca. 96 Stunden betragen, wobei Inkubationszeiten gewählt werden sollten, die eine rasche Auswertung der Ergebnisse ermöglichen (ca. 4 bis 72 Stunden). Wie aber schon weiter oben dargelegt, können die Zellen auch direkt, d. h. ohne Vorinkubation und/oder Stimulation, untersucht werden. Die Auswahl des passenden Verfahrens hängt von der zu untersuchenden Erkrankung, den Mengen an detektierbaren Reaktionen etc. ab. Dem Fachmann ist bekannt, welches Verfahren für eine bestimmte zu treffende Aussage ausgewählt werden muß.

In einer weiteren bevorzugten Ausführungsform werden zur Durchführung des Verfahrens mittels der ELISA-Spot-Methode Mikrotiterplatten verwendet. An den Lochboden der Mikrotiterplatten sind verschiedene stoffspezifische Fremdstoffe fixiert, die dann mit den von den Zellen produzierten Zytokinen spezifisch interagieren, insbesondere diese binden. Bei bekannten Messungen nach der ELISA-Spot-Methode werden pro Loch der Mikrotiterplatte normalerweise eine Million Zellen eingesetzt. Im Blut sind eine Million Zellen gewöhnlich in einem Milliliter Blut enthalten, so daß für eine 96-Loch-Platte schätzungsweise 150 ml Blut erforderlich ist. Es wurde erkannt, daß so große Zellzahlen gar nicht erforderlich sind, weil ein Reader eines automatischen Auswertungsgerätes die Reaktion einzelner Zellen erfassen kann und eine wesentlich geringere Zellzahl ausreicht, um statistisch gesicherte Ergebnisse erhalten zu können. Daher kann die Erfindung den Weg gehen, pro ELISA-Spot, insbesondere pro Loch auf der Mikrotiterplatte, 20.000 bis 200.000 Zellen zu inkubieren. Benutzt man jetzt nicht nur eine, sondern mehrere Löcher, so kann man nach einer statistischen Überprüfung der Ergebnisse auf mehrere Millionen überprüfter Zellen hochrechnen. Mit der Elispot-Methode kann man pro Loch einer 96-Loch-Platte eine positive Zelle auf 200.000 Zellen erfassen und entsprechend, beim Einsatz mehrerer Löcher, die Trefferzahl erhöhen. Da anders als beim ELISA-Test bei der Elispot-Methode die Farbreagenz der Probe nicht löslich vorliegt, sondern sie sich über Stunden auf einen Punkt des Bodens anreichert, der durch eine Farbreaktion definiert werden kann, ist die hohe Sensitivität des Elispots gegeben. Bei der Detektion wird die hohe Empfindlichkeit des Elispots erhalten.

Im Vergleich zum Elispot ist zum Beispiel die Nachweisgrenze beim FACS 200 positive Zellen auf 200.000 eingesetzte Zellen, da beim FACS ca. 0,1% der Zellen positiv sein sollte, damit ein Nachweis erfolgen kann.

In einer weiteren bevorzugten Ausführungsform ist das Farbreagenz daher auch ohne vorhergehende Verdünnung auf die Zellen zu geben. Wie bereits erwähnt, liegt bei der Elispot-Methode das Farbreagenz bzw. der gebundene Farbstoff nicht löslich vor. Als Farbreagenzien/Farbstoffe eignen sich alle Stoffe, die die Interaktion zwischen den von den T-Helferzellen zellulär produzierten Zytokinen und den stoffspezifischen Fremdstoffen nachweisen können, insbesondere ist dabei an Immunfluoreszenzfarbstoffe und Fluorochromfarbstoffe zu denken, die bevorzugt verwendet werden. Die Farbreagenzien/Farbstoffe binden dabei an, bzw. wandern in die evtl. zuvor fixierten Zellen selbst hinein. Dadurch entsteht ein von der Konzentration der von den T-Helferzellen zellulär produzierten Zytokinen abhängiger, örtlich fixierter Farbfleck, der dann mit üblichen Verfahren gemessen und ausgewertet wird.

Weiterhin wird ein Kit offenbart, das mindestens eine Substanz zur Messung der Anzahl an T-Helferzellen und/oder von diesen zellulär produzierten Stoffe, zur Diagnostizierung von Erkrankungen und/oder zur Prognostizierung von deren Verläufen, enthält. Dieses Kit kann insbesondere zur Durchführung eines erfindungsgemäßen Verfahrens verwendet werden. Bei der Substanz, die zur Messung der Anzahl an T-Helferzellen und/oder von diesen zellulär produzierten Stoffe verwendet werden kann, ist insbesondere an Antikörper zu denken. Das Kit wird erfindungsgemäß insbesondere zur Diagnostizierung und/oder Prognostizierung von Infektionserkrankungen, Autoimmunerkrankungen und/oder Allergien verwendet.

Weitere Einzelheiten und Merkmale der Erfindung ergeben sich aus der nachfolgenden Beschreibung der Darstellung des erfindungsgemäßen Verfahrens und von bevorzugten Ausführungsformen in Verbindung mit den Unteransprüchen. Hierbei können die jeweiligen Merkmale für sich alleine oder zu mehreren in Kombination miteinander verwirklicht sein.

### Abbildungen:

- Fig. 1A bis 1 D: Messung von γ-Interferon bei einer Borrelioseinfektion (unterschiedliche Auflösungen).
- Fig. 2A bis 2D: Messung von Interleukin-4 bei einer Borrelioseinfektion (unterschiedliche Auflösungen).
- Fig. 3A bis 3D: Messung von Interleukin-10 bei einer Borrelioseinfektion (unterschiedliche Auflösungen).

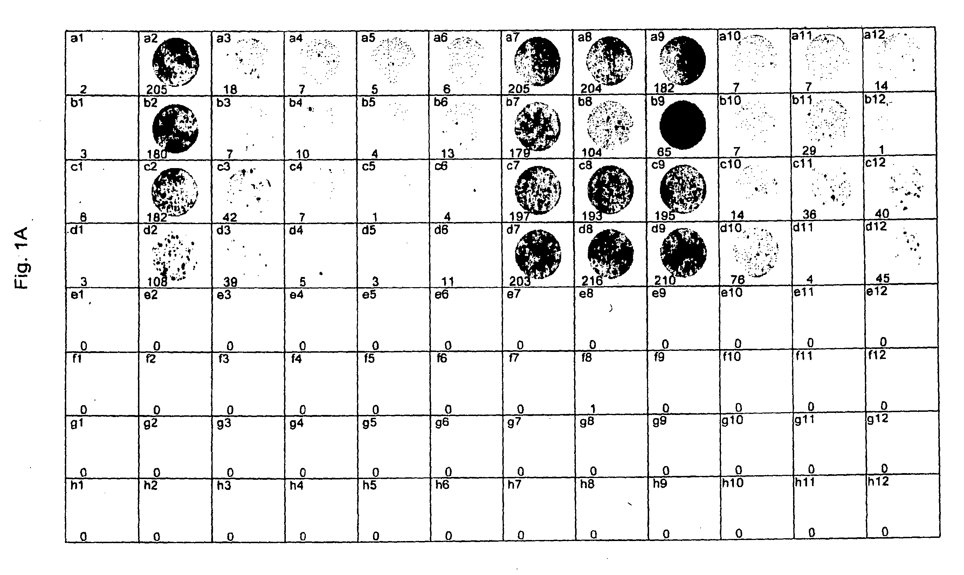

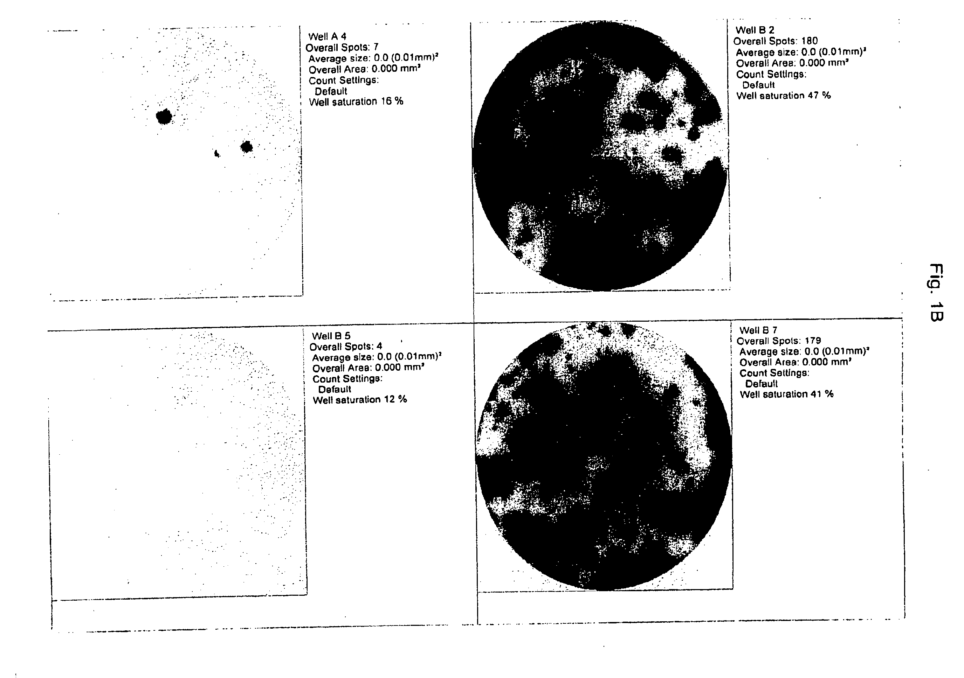

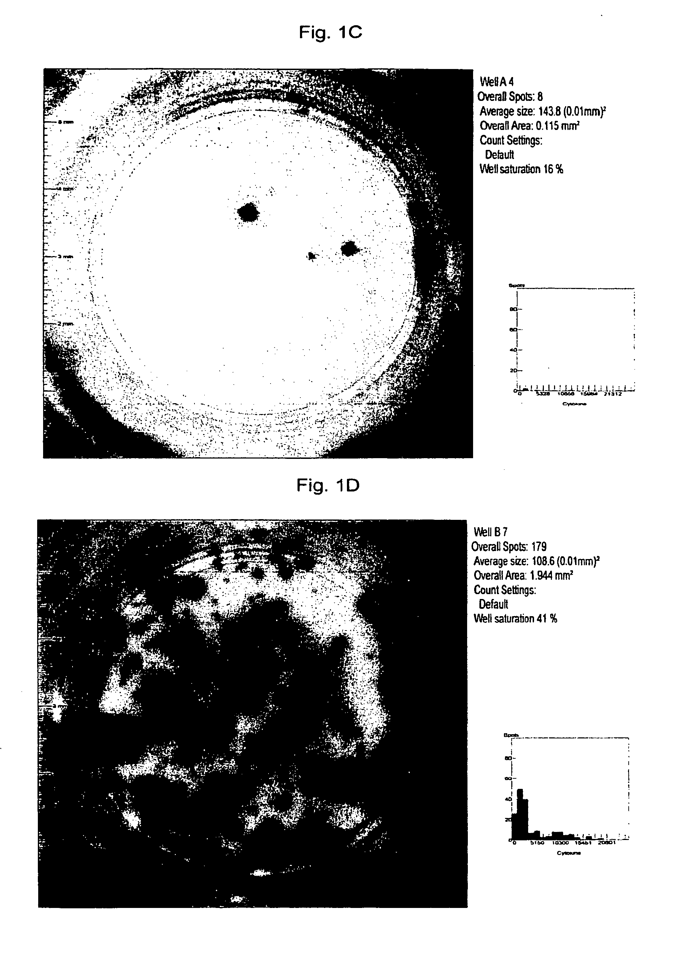

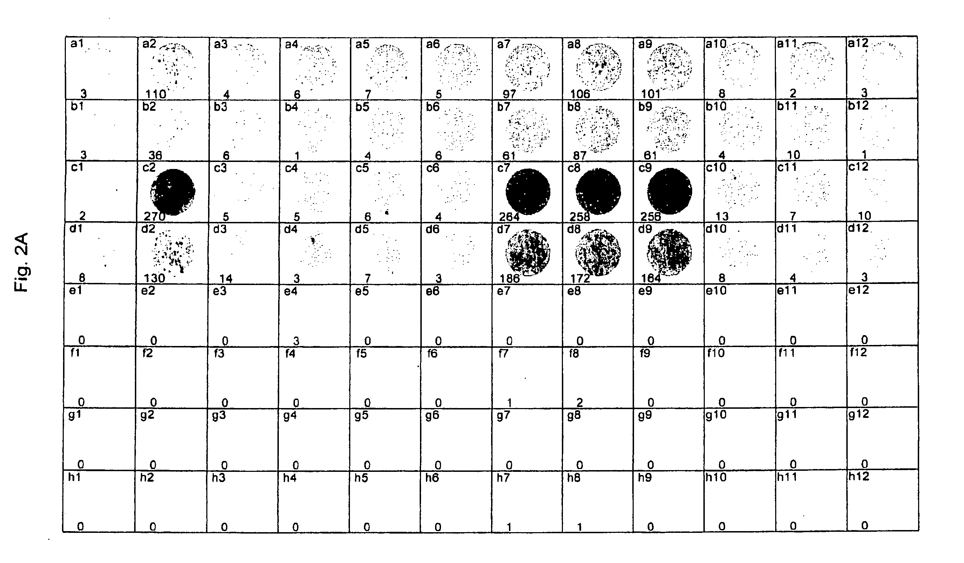

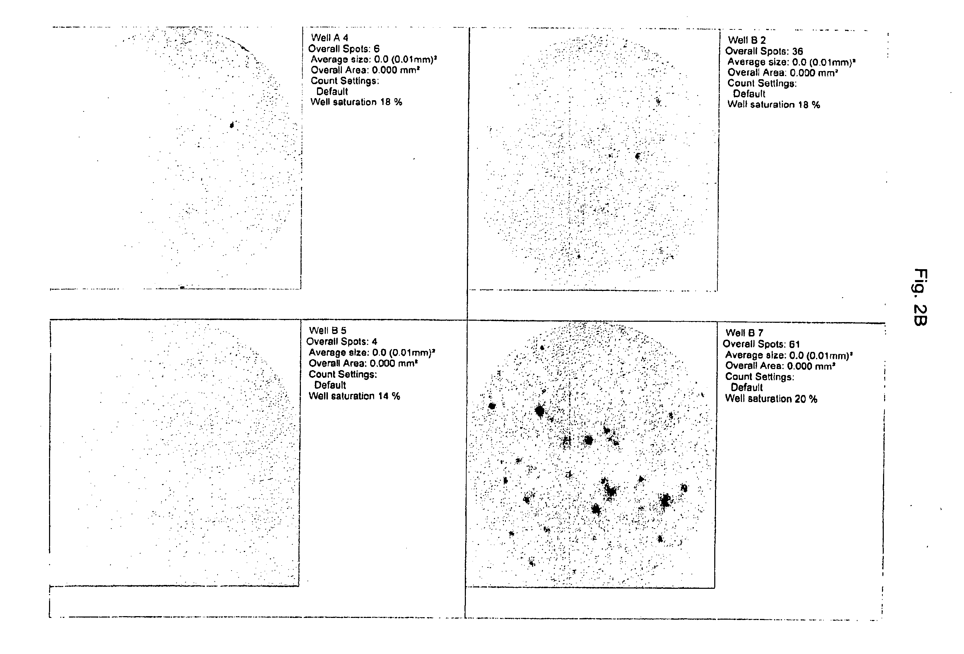

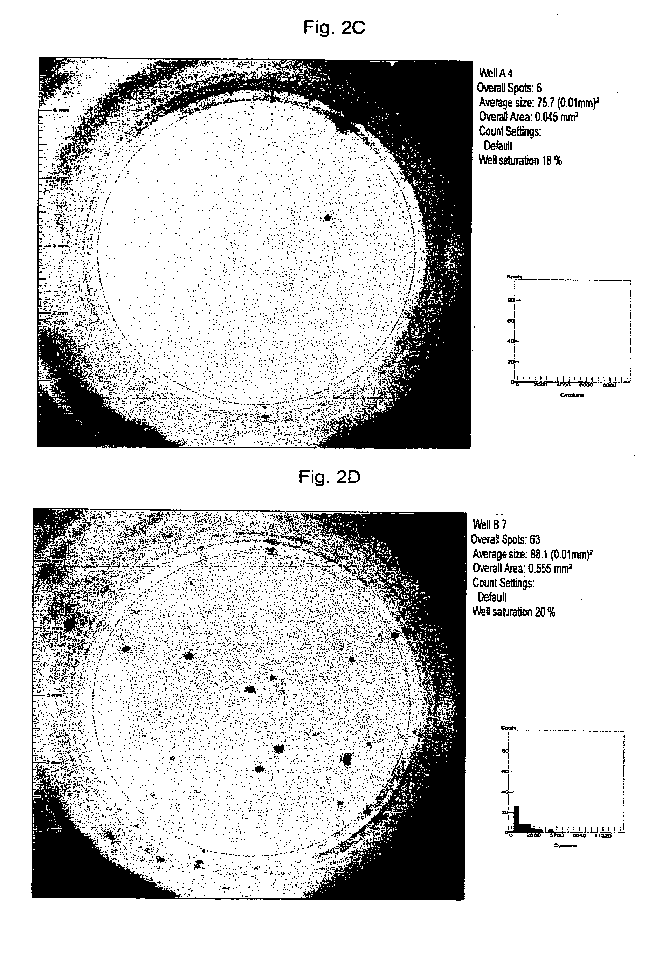

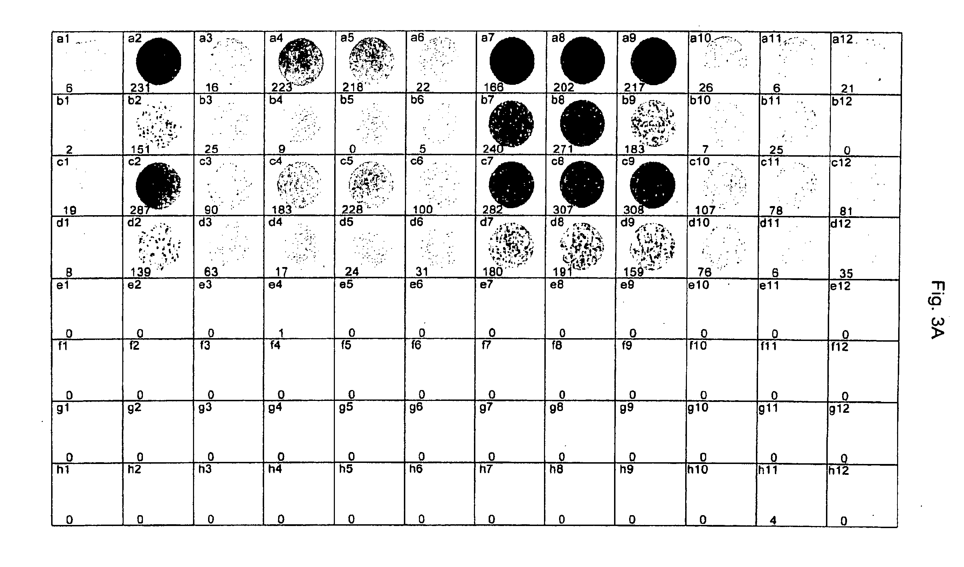

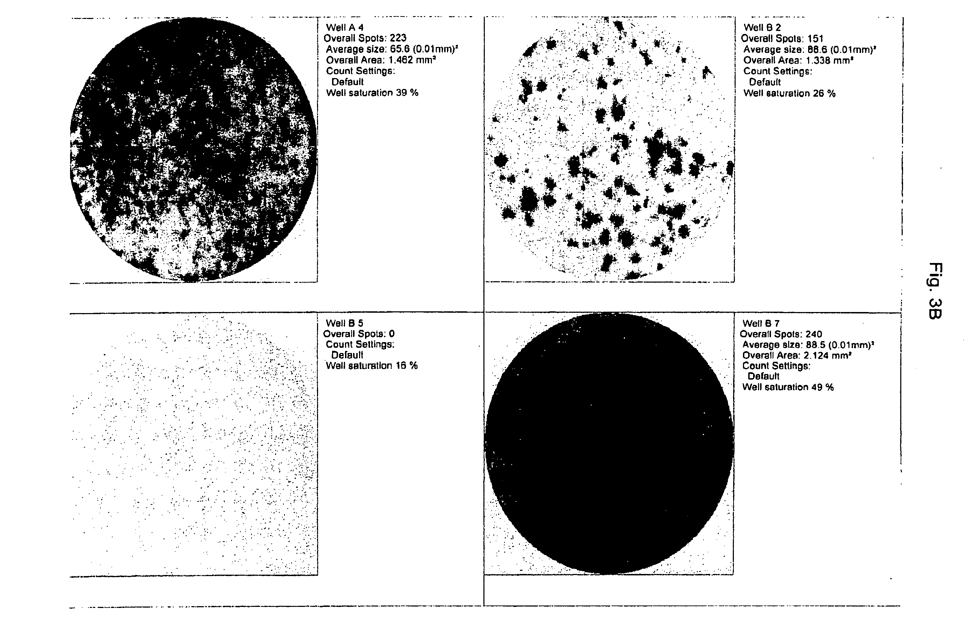

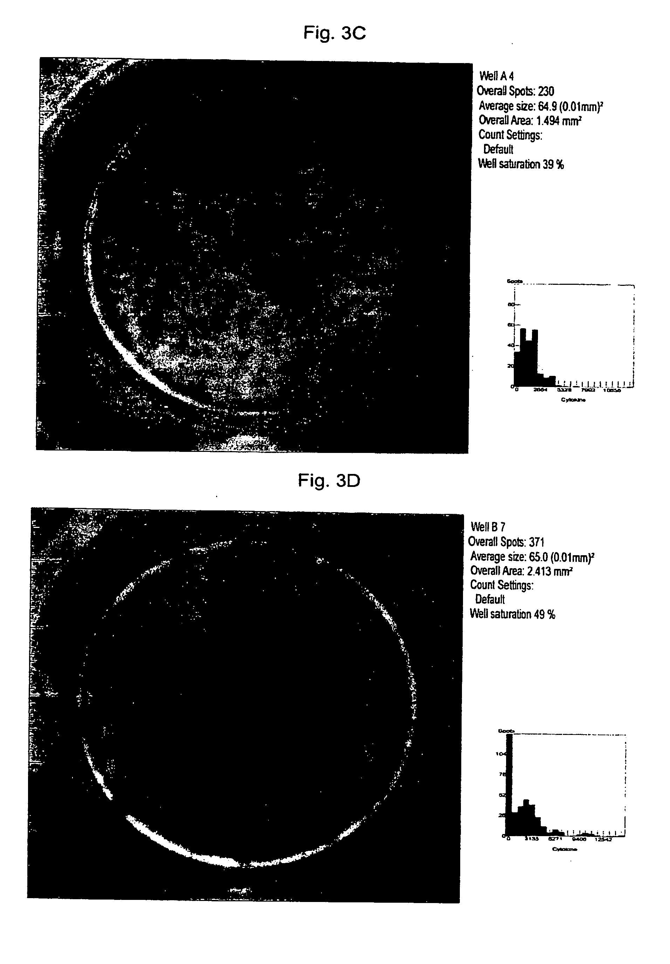

Die Figuren 1A bis 3D zeigen ein typisches Elispot-Protokoll bei einer Borrelioseinfektion. Bezüglich der konkreten Versuchsbedingungen sei auf die Internetseite www.elispot.com verwiesen, wo unter dem Link Scientific Information/Method/ diese beschrieben sind. Verschiedenen Probanden wurde Blut entnommen und verschiedene Markerzytokine bestimmt. In den Fig. 1A - 1D erfolgte die Bestimmung von γ-Interferon als Markerzytokin für T_{H1}-Helferzellen. Dabei stellen die verschiedenen Vertiefungen (well) (a1 bis d12) jeweils verschiedene Probanden dar. In den Figuren 2A bis 2D ist den gleichen Probanden Blut entnommen und auf Interleukin-4 als Markerzytokin für T_{H2}-Helferzellen untersucht worden. Die Vertiefungen a1 bis d12 entsprechen den gleichen Probanden, wie bei der Untersuchung von γ-Interferon. In den Figuren 3A bis 3D ist erneut den gleichen Probanden Blut entnommen und auf Interleukin-10 als Markerzytokin für T_{H2}-Helferzellen untersucht worden. Betrachtet man nun beispielsweise die Vertiefung a4, so stellt man vergleichsweise wenig γ-Interferon (z. B. Fig. 1C) mit moderater Intensität fest. Wird nun als Markerzytokin für T_{H2}-Helferzellen II-4 gemessen (Fig. 2A - 2D), so stellt man kaum II-4 Spots (z. B. Fig. 2C) bei dem Probanden a4 fest. Somit ist das Verhältnis T_{H1}-:T_{H2}-Helferzellen eher groß, womit theoretisch eine gute Prognose verbunden wäre. Allerdings ist zu berücksichtigen, daß die Messung von II-4 kaum verwertbare Spots ergab, so daß kaum statistisch relevante Aussagen zu machen sind. Wird der gleiche Proband auf Interleukin-10 gemessen (Fig. 3A bis 3D), so ergibt sich ein gänzlich anderes Bild. Es kann sehr viel II-10 nachgewiesen werden (z. B. Fig. 3C), womit das Verhältnis TH₁-:T_{H2}-Helferzellen klein ausfällt und sich eine schlechte Prognose für den Probanden ergibt.

Es ist offensichtlich, daß das Verhältnis von γ-Interferon zu Interleukin-4 nicht aussagekräftig ist, da Interleukin-4 nicht ausreichend bei den entsprechenden Probanden gebildet wurde. Erst der Vergleich von γ-Interferon mit Interleukin-10 läßt eine aussagefähige Wertung zu.

Dieselben Experimente wurden bei verschiedenen anderen Erkrankungen durchgeführt und es ergaben sich nachfolgende Ergebnisse. Generell wurden diese Experimente immer mit spezifischen Antigenen und/ oder Antikörpern gegen die zu untersuchende Erkrankung durchgeführt, um nur spezifische T-Helferzellen und/oder von diesen zellulär produzierten Stoffe in die Untersuchungen aufzunehmen. Als Markerzytokine dienten im wesentlichen γ-Interferon (T_{H1}-Zellen) bzw. Interleukin-10 (T_{H2}-Zellen).

### Infektionserkrankungen:

### Borreliose:

Borreliose: Monitoring und Prognose von Borreliosen (Infektionen mit Borrelia burgdorferi). Hierbei wird die Borreliose anhand von borrelienspezifischen Antigenen wie z.B. OSP A, OSP C, innerFlagelinfragment oder anderen, von verschiedenen Borrelienstämmen stammenden Antigenen (z.B. Borrelia burgdorferi, Borrelia afzelli bzw. Borrelia garinii), gemessen. Als Marker Zytokin für T_{H1}-Helferzellen dient hierbei das γ-Interferon, als Marker für T_{H2}-Helferzellen Interleukin-10. Es wurde gefunden, daß ein Verhältnis größer 1:5 (T_{H1}:T_{H2}), eher zu einem chronischen Verlauf bei dieser Erkrankung führt. So wurde z. B. konkret bei einem Probanden 30 γ-Interferon zu 400 Interleukin-10 gemessen (siehe auch Fig. 1A - 3D).

### CMV:

CMV tritt vor allem nach Transplantationen bzw. nach Zugabe von Immunsuppressiva auf. Es wurde nun gefunden, daß ein Verhältnis kleiner 1:5 (T_{H1}:T_{H2}) eine gute Prognose darstellt. Konkret wurde ein Proband nach einer Transplantation auf seinen T_{H1}- und T_{H2}-Zelltiter (γ-Interferon/ Interleukin-10; kommerziell erhältliche Antikörper gegen CMV; Nur Markerzytokine aus antigenspezifischen Zellen). Nach 60 Tagen zeigte der Proband 40 γ-Interferon-Spots und 100 Interleukin-10-Spots (1:2,5), was für eine eher gute Prognose spricht.

Bei einem anderen Probanden ergab sich nach einer Transplantation folgendes Bild. Sechzig Tage nach Transplantation wurden 30 γ-Interferon-Spots zu 200 Interleukin-10-Spots (1:6,6) gemessen, was keine gute Prognose darstellt.

Ähnliche Ergebnisse wurden mit EBV (Eppstein-Barr-Virus) und Herpesviren erzielt.

### Hepatitis C:

Es wurde gefunden, daß ein Verhältnis 1:2 und weniger für eine gute Prognose spricht. Bei einem Probanden wurden 40 γ-Interferon zu 80 Interleukin-10-Spots (Verhältnis 1:2) gemessen. Bei diesem Probanden ist keine Therapie notwendig, da die Erkrankung von selbst ausheilt. Bei einem weiteren Probanden wurden 15 γ-Interferon und 150 Interleukin-10-Spots (Verhältnis 1:10) gemessen, was eine extrem schlechte Prognose für die Zukunft befürchten läßt. Hier besteht eine Tendenz zu Leberzirrhose sowie Leberfibrose. Eine Therapie ist angezeigt.

### Autoimmunerkrankungen:

### Rheuma und Morbus-Crohn:

Es wurde gefunden, daß ein T_{H1}- zu T_{H2}-Verhältnis von 1:2 und größer (gemessen anhand der Spots von γ-Interferon und Interleukin-10) eher eine schlechte Prognose darstellt. Eine Therapie ist nötig.

### Allergien:

Hierbei wurde bei Hyposensibilisierungsprognosen gefunden, daß ein Verhältnis von TH₁ zu T_{H2} von größer 1:2 eine eher schlechte Prognose darstellt.

Allgemein konnte mit den Versuchen gezeigt werden, daß die Anzahl an γ-Interferon (oder anderer Markerstoffe für T_{H1}-Helferzellen) alleine nicht entscheidend für die Diagnose oder Prognose der Erkrankung sind. Mit diesen Ergebnissen alleine kann man keine Aussage dahingehend treffen, ob ein guter Verlauf, d. h. eine Selbstheilung, erfolgt oder nicht. Entscheidend ist immer das Verhältnis der von den T-Helferzellen produzierten Zytokine zueinander, d. h. das Verhältnis der Anzahl und der Aktivität der Reaktion. Vermeintlich viele γ-Interferon-Spots (oder andere T_{H1}-Helferzellenmarkerstoffe) alleine sind nicht aussagekräftig, da eventuell die Menge an T_{H2}-Helferzellenmarkerstoffe wie z. B. Interleukin-10 höher sein kann und somit eine im Endeffekt schlechte Prognose darstellt. Weiterhin konnte festgestellt werden, daß die Wirkung der Immunreaktion nicht alleine abhängig von der Anzahl der Reaktionen, sondern vielmehr auch von deren Aktivitäten/Intensitäten sowie des Verhältnisses von TH₁ zu T_{H2} ist.

## Patentansprüche

1. Verfahren zur Diagnostizierung von Infektionserkrankungen und/oder zur Prognostizierung von deren Verläufen, wobei die Anzahl von T_{H1}- und T_{H2}-Zellen (T-Helferzellen) produzierten Zytokinen in vom lebenden Organismus entnommenen menschlichen oder tierischen Zellen gemessen wird, wobei die Zytokine in Gegenwart von stoffspezifischen Fremdstoffen inkubiert werden, wobei die Zytokine mit den stoffspezifischen Fremdstoffen interagieren, diese Interaktion durch eine Farbreaktion nach der ELISA-Spot-Methode nachgewiesen wird und anschließend das Verhältnis der Zytokine bestimmt wird, **dadurch gekennzeichnet, dass** es sich bei den von T_{H1-}Zellen produzierten Zytokinen um γ-Interferon und bei den von T_{H2}-Zellen produzierten Zytokinen um Interleukin-10 handelt und das Verhältnis von γ-Interferon zu Interleukin-10 bestimmt wird.

2. Verfahren nach Anspruch 1, **dadurch gekennzeichnet, daß** es sich bei den menschlichen oder tierischen Zellen um Blutzellen handelt, wobei vorzugsweise die Blutzellen vor der Messung der von den T-Helferzellen produzierten Zytokine angereichert werden.

3. Verfahren nach Anspruch 1 oder 2, **dadurch gekennzeichnet, daß** es sich bei den Infektionserkrankungen um Borreliose, CMV (Zytomegalie-Virus), EBV (Eppstein-Barr-Virus), Herpes, Hepatitis C und/oder Tuberkulose handelt.

4. Verfahren nach einem der vorhergehenden Ansprüche, **dadurch gekennzeichnet, daß** es sich bei den stoffspezifischen Fremdstoffen um Capture-Moleküle, insbesondere um Antigene und/oder Antikörper, handelt.

5. Verfahren nach einem der vorhergehenden Ansprüche, **dadurch gekennzeichnet, daß** die von den T-Helferzellen produzierten Zytokine mit verschiedenen stoffspezifischen Fremdstoffen spezifisch interagieren, vorzugsweise spezifisch mit diesen binden, wobei vorzugsweise die spezifischen Interaktionen zu mindestens zwei, vorzugsweise einer Vielzahl, an Farbreaktionen führen und diese nach Art der Farbe getrennt erfaßt und ausgewertet werden.

6. Verfahren nach einem der vorhergehenden Ansprüche, **dadurch gekennzeichnet, daß** die Zellen in einem Untersuchungsgefäß, insbesondere in den Löchern von Mikrotiterplatten, fixiert werden.

7. Verfahren nach einem der vorhergehenden Ansprüche, **dadurch gekennzeichnet, daß** die Zellen vor der Messung der zellulär produzierten Zytokine lysiert werden.

8. Verfahren nach einem der vorhergehenden Ansprüche, **dadurch gekennzeichnet, daß** die Zellen in einem stimulierten Zustand inkubiert werden, insbesondere daß die Zellen *ex vivo* stimuliert werden, wobei vorzugsweise die Zellen mit einem Antigen und/oder Mitogen stimuliert werden.

9. Verfahren nach Anspruch 8, **dadurch gekennzeichnet, daß** durch die Stimulierung eine Vermehrung der von den T-Helferzellen produzierten Zytokine erfolgt.

10. Verfahren nach einem der vorhergehenden Ansprüche, **dadurch gekennzeichnet, daß** zur Durchführung der Messung der Anzahl der von den T-Helferzellen produzierten Zytokine mittels der ELISA-Spot-Methode Mikrotiterplatten verwendet werden.

11. Verfahren nach einem der vorhergehenden Ansprüche, **dadurch gekennzeichnet, daß** ein Farbreagenz ohne vorhergehende Verdünnung auf die Zellen gegeben wird.

## Claims

1. A method for diagnosis of infectious diseases and/or for prognosis of disease states, with the number of cytokines produced by T_{H1} and T_{H2} cells (T helper cells) being monitored in human or animal cells derived from a living organism, the cytokines being incubated in the presence of substance-specific impurities, the cytokines interacting with the substance-specific impurities, said interaction being detected by a color reaction according to the ELISA spot method, and subsequently the ratio of the cytokines being determined,
**characterized in that**
the cytokines produced by T_{H1} cells are interferon gamma (IFNγ) and the cytokines produced by TH₂ cells are interleukin-10 (IL-10) and the ratio of interferon gamma to interleukin-10 is determined.

2. The method according to claim 1, **characterized in that** the human or animal cells are blood cells, wherein preferably the blood cells are accumulated prior to determination of the cytokines produced by the T helper cells.

3. The method according to claim 1 or 2, **characterized in that** the infectious diseases are borreliosis, CMV (cytomegalovirus infection), EBV (Epstein Barr virus), herpes, hepatitis C and/or tuberculosis.

4. The method according to any one of the preceding claims, **characterized in that** the substance-specific impurities are capture molecules, in particular antigens and/or antibodies.

5. The method according to any one of the preceding claims, **characterized in that** the cytokines produced by the T helper cells are capable of specifically interacting with different substance-specific impurities, preferably capable of specifically binding to said impurities, wherein preferably the specific interactions result in at least two, preferably a plurality of color reactions, and said color reactions are detected and evaluated selectively according to the type of color.

6. The method according to any one of the preceding claims, **characterized in that** the cells are fixed in a test vessel, in particular in the wells of microtiter plates.

7. The method according to any one of the preceding claims, **characterized in that** the cells are lysed prior to measurement of the cellular produced cytokines.

8. The method according to any one of the preceding claims, **characterized in that** the cells are incubated in a stimulated condition, in particular **in that** the cells are stimulated *ex vivo*, wherein preferably the cells are stimulated using an antigen and/or mitogen.

9. The method according to claim 8, **characterized in that** due to the stimulation, there is an increase in the cytokines produced by the T helper cells.

10. The method according to any one of the preceding claims, **characterized in that** microtiter plates are used for performing a measurement of the number of cytokines produced by the T helper cells by means of the ELISA spot method.

11. The method according to any one of the preceding claims, **characterized in that** a color reagent is added to the cells without previous dilution.

## Revendications

1. Procédé pour diagnostiquer des maladies infectieuses et/ou pour pronostiquer leur évolution, dans lequel le nombre de cytokines produites par des cellules T_{H1} et T_{H2} (cellules T auxiliaires) est mesuré dans des cellules humaines ou animales prélevées de l'organisme vivant, les cytokines étant incubées en présence de substances étrangères spécifiques, les cytokines interagissant avec les substances étrangères spécifiques, cette interaction étant détectée par une réaction colorée par la méthode ELISA-Spot, ce après quoi on détermine la proportion des cytokines, **caractérisé en ce que**, pour ce qui concerne les cytokines produites par les cellules T_{H1}, d'interféron γ et, pour ce qui concerne les cytokines produites par les cellules T_{H2}, d'interleukine 10 et que l'on détermine le rapport de l'interféron γ à l'interleukine 10.

2. Procédé selon la revendication 1, **caractérisé en ce que**, pour ce qui concerne les cellules humaines ou animales, il s'agit de cellules sanguines, les cellules sanguines étant de préférence enrichies avant la mesure des cytokines produites par les cellules T auxiliaires.

3. Procédé selon la revendication 1 ou 2, **caractérisé en ce que**, pour ce qui concerne les maladies infectieuses, il s'agit de la borréliose, du CMV (virus de la cytomégalie), de l'EBV (virus Eppstein-Barr), de l'herpès, de l'hépatite C et/ou de la tuberculose.

4. Procédé selon l'une des revendications précédentes, **caractérisé en ce que**, pour ce qui concerne les substances étrangères spécifiques, il s'agit de molécules de capture, en particulier d'antigènes et/ou d'anticorps.

5. Procédé selon l'une des revendications précédentes, **caractérisé en ce que** les cytokines produites par les cellules T auxiliaires interagissent d'une manière spécifique avec différentes substances étrangères spécifiques, se lient de préférence d'une manière spécifique à ces dernières, de préférence les interactions spécifiques conduisant à au moins deux, de préférence à un grand nombre de réactions colorées, ces dernières étant détectées et évaluées séparément selon la nature de la couleur.

6. Procédé selon l'une des revendications précédentes, **caractérisé en ce que** les cellules sont fixées dans un récipient pour essais, en particulier dans les puits de plaques de microtitrage.

7. Procédé selon l'une des revendications précédentes, **caractérisé en ce que** les cellules sont lysées avant la mesure des cytokines produites par des cellules.

8. Procédé selon l'une des revendications précédentes, **caractérisé en ce que** les cellules sont incubées dans un état stimulé, en particulier que les cellules sont stimulées *ex vivo,* les cellules étant de préférence stimulées avec un antigène et/ou un mitogène.

9. Procédé selon la revendication 8, **caractérisé en ce que** la stimulation provoque une multiplication des cytokines produites par les cellules T auxiliaires.

10. Procédé selon l'une des revendications précédentes, **caractérisé en ce que**, pour mettre en oeuvre la mesure du nombre des cytokines produites par les cellules T auxiliaires par la méthode ELISA-Spot, on utilise des plaques de microtitrage.

11. Procédé selon l'une des revendications précédentes, **caractérisé en ce qu'**un réactif coloré est placé sur les cellules sans dilution préalable.
